# EUROPÄISCHE PATENTSCHRIFT

(11) **EP 1 392 667 B1**
(45) Veröffentlichungstag und Bekanntmachung des Hinweises auf die Patenterteilung: **27.06.2007**
(21) Anmeldenummer: 02743054.5
(22) Anmeldetag: 17.05.2002
(51) Int. Cl.: C07D 295/08

(54) **VERFAHREN ZUR HERSTELLUNG VON BIPERIDEN**
METHOD FOR THE PRODUCTION OF BIPERIDIN
PROCEDE DE PRODUCTION DE BIPERIDENE

(30) Priorität: 18.05.2001 DE 10124451
(43) Veröffentlichungstag der Anmeldung: 03.03.2004
(73) Patentinhaber: LABORATORIO FARMACEUTICO S.I.T. SPECIALITA' IGIENICO TERAPEUTICHE S.r.l., 27035 Mede (Pavia) (IT)
(72) Erfinder: KLEIN, Peter, 67134 Birkenheide (DE); THYES, Marco, 67061 Ludwigshafen (DE); GROSSE, Markus, 68723 Schwetzingen (DE); WEBER, Klaus, Dieter, 67346 Speyer (DE)
(74) Vertreter: De Gregori, Antonella
(86) Internationale Anmeldenummer: PCT/EP2002/005496
(87) Internationale Veröffentlichungsnummer: WO 2002/094800

(56) Entgegenhaltungen:
- DE-B- 1 005 067
- US-A- 2 789 110
- R. BRESLOW ET AL: "In the origin of product selectivity in aqueous Diels-Alder reactions" TETRAHEDRON LETTERS, Bd. 25, Nr. 12, 1984, Seiten 1239-40, XP002212222 in der Anmeldung erwähnt

## Beschreibung

Die vorliegende Erfindung betrifft ein Verfahren zur Herstellung von Biperiden.

Biperiden ist ein bekanntes zentrales Anticholinergikum und wird zur Behandlung der Parkinsonschen Krankheit eingesetzt (Ullmanns Enzyklopädie der technischen Chemie, 4. Aufl., Band 21, Verlag Chemie, 1982, S. 627). Es handelt sich um ein Racemat aus 1-(Bicyclo[2.2.1]hept-5-en-2-yl(exo,R))-1-phenyl-3-piperidinopropanol(1,S) und 1-(Bicyclo[2.2.1]hept-5-en-2-yl(exo,S))-1-phenyl-3-piperidinopropanol(1,R) (Ia) und stellt eines von vier möglichen Enantiomerenpaaren (Ia-d) des Aminoalkohols 1-(Bicyclo[2.2.1]hept-5-en-2-yl)-1-phenyl-3-piperidino-1-propanol (I) dar.

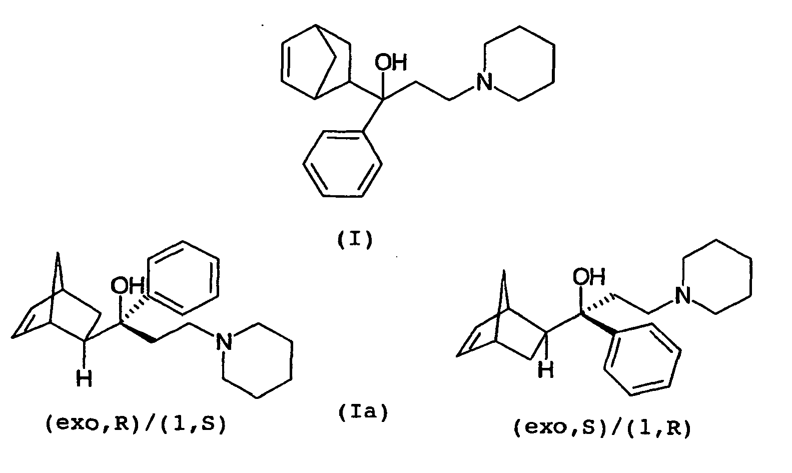

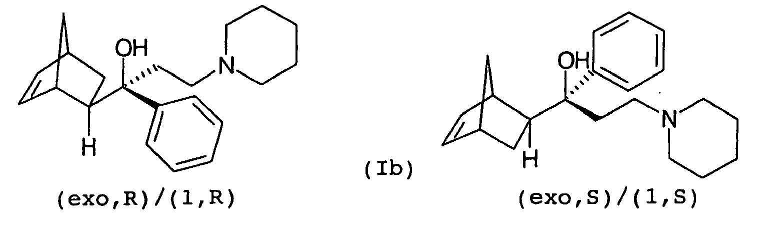

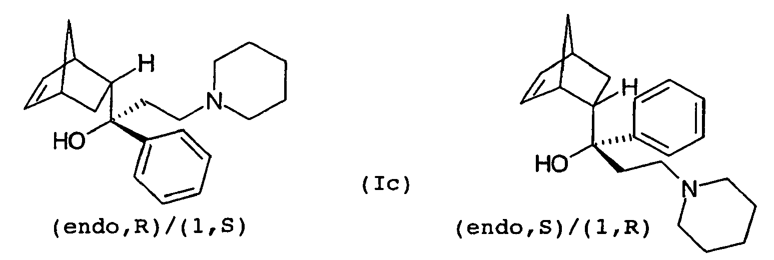

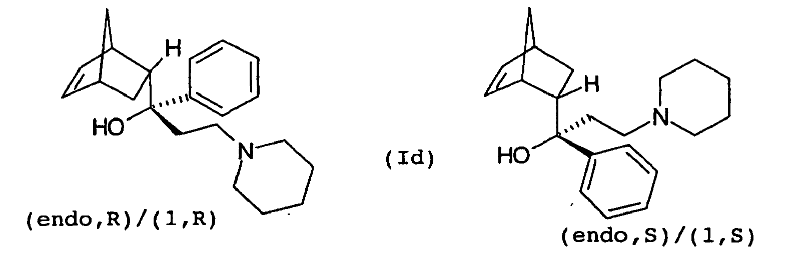

Die DE 1 005 067 und die US 2,789,110 beschreiben die Herstellung des Aminoalkohols I durch Umsetzung von 1-(Bicyclo[2.2.1]hept-5-en-2-yl)-3-piperidino-1-propanon (II) mit einem Phenylmagnesiumhalogenid. Die US 2,789,110 beschreibt zudem die Herstellung des Propanons II, ausgehend von 1-(Bicyclo[2.2.1]hept-5-en-2-yl)ethanon (III), Paraformaldehyd und Piperidinhydrochlorid in einer Reaktion nach Mannich sowie die Herstellung des Ethanons III aus Cyclopentadien und Methylvinylketon in einer Cycloaddition nach Diels und Alder.

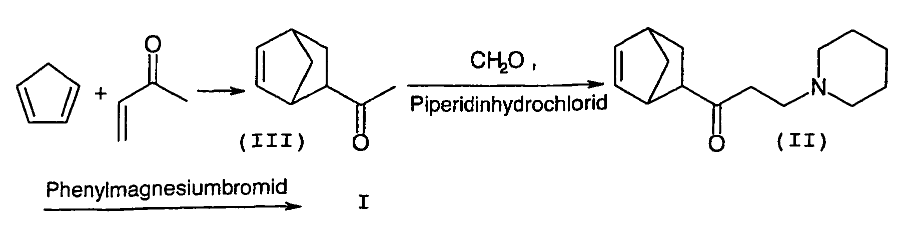

Weder die DE 1 005 067 noch die US 2,789,110 offenbaren, ob es sich bei dem so erhaltenen Aminoalkohol I um ein Isomerengemisch oder um ein reines Isomer handelt.

Das Edukt der Propanolherstellung, 1-(Bicyclo[2.2.1]hept-5-en-2-yl)-3-piperidino-l-propanon (II), kann in zwei isomeren Formen, als exo- oder als endo-Isomeres (II-exo, II-endo), vorliegen, wobei nur die exo-Form in der oben genannten Reaktion mit einem Phenylmagnesiumhalogenid Biperiden ergeben kann.

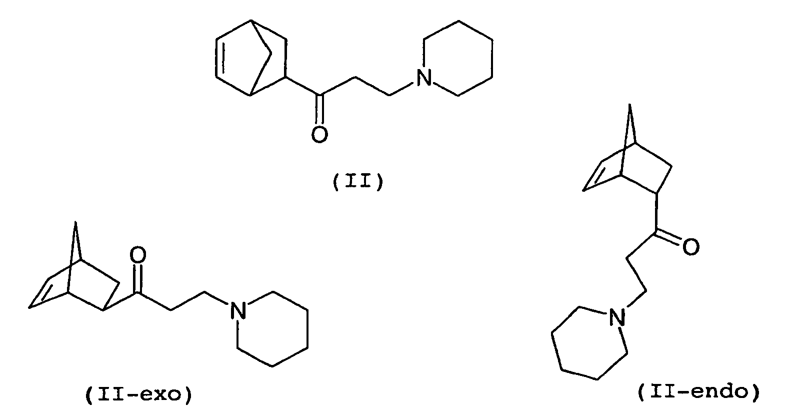

Die Strukturformeln des II-exo und des II-endo zeigen der Einfachheit halber jeweils nur eines von zwei möglichen Enantiomeren des exo- bzw. endo-Isomers. Im Folgenden bezieht sich die Bezeichnung II-exo bzw. II-endo jedoch auf das Enantiomerenpaar der exo- bzw. endo-Form.

Auch 1-(Bicyclo[2.2.1]hept-5-en-2-yl)ethanon (III), die Ausgangssubstanz für die Synthese des Propanons II, kann sowohl als exoals auch als endo-Isomeres vorliegen (III-exo, III-endo) und entsprechend führt nur die Umsetzung des exo-Isomeren in den Folgeschritten zu Biperiden.

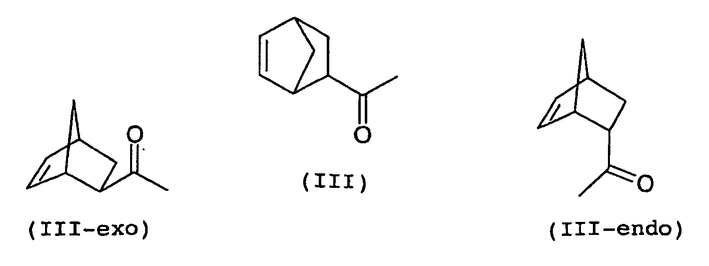

Die Strukturformeln des III-exo und des III-endo zeigen der Einfachheit halber jeweils nur eines von zwei möglichen Enantiomeren des exo- bzw. endo-Isomers. Im Folgenden bezieht sich die Bezeichnung III-exo bzw. III-endo jedoch auf das Enantiomerenpaar der exo- bzw. endo-Form.

Keiner der oben genannten Schriften lassen sich Angaben zur Konfiguration der eingesetzten Edukte III und Zwischenprodukte II entnehmen.

Es ist bekannt, dass das 1-(Bicyclo[2.2.1]hept-5-en-2-yl)ethanon (III) aus der Cycloaddition in einem exo/endo-Verhältnis von 1:4 erhalten wird (z.B. R. Breslow, U. Maitra, Tetrahedron Letters, 1984, 25, 1239). Da der eingangs genannte Stand der Technik keinerlei Angaben zur Stereochemie des Ethanons III macht, ist davon auszugehen, dass das Ethanon III in diesem Isomerenverhältnis zur Herstellung des Aminoalkohols I eingesetzt wurde.

Die Herstellung von exo-1-(Bicyclo[2.2.1]hept-5-en-2-yl)ethanon (III-exo) ist 1965 von J.G. Dinwiddie und S.P. McManus beschrieben worden (J. Org. Chem., 1965, 30, 766). Dabei werden exo/endo-Gemische von 1-(Bicyclo[2.2.1]hept-5-en-2-yl)ethanon (III), in denen der endo-Anteil überwiegt, in Methanol in Gegenwart von Natriummethanolat erhitzt und isomerisieren zu Gemischen mit einem exo-Anteil von ca. 70 %. Aus diesen kann durch fraktionierte Destillation und gegebenenfalls Redestillieren des Destillats exo-1-(Bicyclo[2.2.1]hept-5-en-2-yl)ethanon (III-exo) mit einem Reinheitsgrad von bis zu 95 % erhalten werden.

Versuche der Anmelderin haben gezeigt, dass auch bei der Verwendung von nahezu einheitlichem exo-1-(Bicyclo[2.2.1]hept-5-en-2-yl)ethanon (III-exo), d. h. von einem Ethanon III mit einem exo-Anteil von wenigstens 95 %, als Ausgangsmaterial sauberes Biperiden (Ia) in nur geringen Ausbeuten erhalten werden kann, wobei sowohl die Umsetzung des 1-(Bicyclo[2.2.1]hept-5-en-2-yl)-3-piperidino-1-propanons (II) mit einem Phenylmagnesiumhalogenid als auch die Isolierung des Biperidens (Ia) aus dem Isomerengemisch des bei dieser Umsetzung gebildeten Aminoalkohols I mit einer schlechten Ausbeute an Biperiden (Ia) verlaufen. Unter sauberem Biperiden versteht man ein Biperiden (Ia) mit einer Reinheit von wenigstens 99,0 %, wie sie für pharmazeutische Anwendungen in der Regel erforderlich ist.

Der vorliegenden Erfindung liegt die Aufgabe zugrunde, ein Verfahren zur Herstellung von Biperiden (Ia) bereit zu stellen, das dieses in einer höheren Ausbeute liefert.

Die Aufgabe konnte gelöst werden durch ein Verfahren zur Herstellung von Biperiden durch Umsetzung von 1-(Bicyclo[2.2.1]hept-5-en-2-yl)-3-piperidino-1-propanon (II) mit einem exo/endo-Verhältnis von wenigstens 2,5:1 mit einer Phenylmagnesiumverbindung zu einem Biperiden (Ia) enthaltenden Isomerengemisch von 1-(Bicyclo-[2.2.1]hept-5-en-2-yl)-1-phenyl-3-piperidino-1-propanol (I), dadurch gekennzeichnet, dass die Isolierung des Biperidens (Ia) aus dem Isomerengemisch folgende Schritte umfasst:
a) Umsetzung des Isomerengemischs mit Chlorwasserstoff in einem Gemisch aus Wasser und einem polaren, mit Wasser begrenzt oder vollständig mischbaren organischen Lösungsmittel und Isolierung des dabei gebildeten Hydrochlorids,
b) Umsetzung des Hydrochlorids in einer Mischung aus Wasser und wenigstens einem polaren, mit Wasser begrenzt oder nicht mischbaren Dialkylether mit 4 bis 8 C-Atomen mit einer Base,
c) Trennen der zwei gebildeten Phasen in der Wärme,
d) Verdampfen eines Teils des Ethers aus der organischen Phase und
e) Kristallisieren des Biperidens durch Abkühlen.

Bei den im erfindungsgemäßen Verfahren eingesetzten exo- und endo-Isomeren handelt es sich, wie bereits für das exo- und endo-Ethanon III-exo und III-endo sowie für das exo- und endo-Propanon II-exo und II-endo beschrieben, um Enantiomerenpaare. Um zu Biperiden (Ia) zu gelangen, das selbst ein Racemat ist, werden racemische Enantiomerengemische der Ausgangsstoffe und der Zwischenprodukte eingesetzt. Das erfindungsgemäße Verfahren kann jedoch auch auf reine Enantiomere sowie auf nicht racemische Enantiomerengemische angewendet werden.

Die Umsetzung des Propanons II mit einer geeigneten Phenylmagnesiumverbindung erfolgt in der Regel in einem geeigneten Lösungsmittel. Geeignete Phenylmagnesiumverbindungen sind Phenylmagnesiumhalogenide, beispielsweise Phenylmagnesiumchlorid oder Phenylmagnesiumbromid, Diphenylmagnesium und Phenylmagnesiumalkoxide der allgemeinen Formel IV 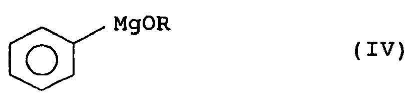 worin R für C₁-C₄-Alkyl, wie Methyl, Ethyl, n-Propyl, Isopropyl oder n-Butyl, C₄-C₆-Cycloalkyl, wie Cyclohexyl, C₄-C₆-Cycloalkyl-C₁-C₄-alkyl, wie 2-Cyclohexylethyl, Phenyl-C₁-C₄-alkyl, wie Benzyl, 2-Phenylethyl oder 3-Phenylpropyl, substituiertes Phenyl-C₁-C₄-alkyl, wie 3,4-(Methylendioxy)benzyl, Heteroaryl, wie 8-Chinolyl, Heteroaryl-C₁-C₄-alkyl, wie Furfuryl, 2-Thienylmethyl oder 2-(2-Thienyl)ethyl, oder Benzhydryl steht. Vorzugsweise wird Diphenylmagnesium und insbesondere das Phenylmagnesiumalkoxid verwendet.

Geeignete Lösungsmittel für die Umsetzung von II mit der Phenylmagnesiumverbindung sind Aromaten wie Benzol, Toluol oder Xylole, acyclische oder cyclische Ether mit 4 bis 6 C-Atomen, Gemische davon oder deren Mischungen mit aliphatischen oder alicyclischen Kohlenwasserstoffen wie n-Hexan bzw. Cyclohexan. Geeignete alicyclische Ether sind z. B. Diethylether und tert-Butylmethylether, geeignete cyclische Ether sind z. B. Tetrahydrofuran und Dioxan. Bevorzugt verwendet man Diethylether, Tetrahydrofuran oder Dioxan oder Mischungen davon. Die Lösungsmittel werden in der Regel, wie für Grignard-Reaktionen üblich, wasserfrei eingesetzt.

Das Phenylmagnesiumalkoxid IV wird in allgemein bekannter Weise, z. B. durch Umsetzung von Diphenylmagnesium mit einem Alkohol der allgemeinen Formel ROH, worin R wie oben definiert ist, hergestellt. Diphenylmagnesium und der Alkohol werden dabei in einem Molverhältnis im Bereich von 1:0,9 bis 1:1,5, vorzugsweise im Bereich von 1:1 bis 1:1,2 und besonders bevorzugt etwa äquimolar umgesetzt. Gewöhnlich wird Diphenylmagnesium, das üblicherweise wie weiter unten beschrieben in situ erzeugt wird, in einem der oben genannten, für Grignard-Reaktionen geeigneten Lösungsmittel vorgelegt und der Alkohol in der Regel portionsweise über eine Zeitspanne von 5 Minuten bis etwa eine Stunde bei einer Temperatur von 0 bis 80 °C, vorzugsweise von 0 bis 50 °C und besonders bevorzugt von 0 bis 40 °C zugegeben. Nach beendeter Zugabe kann man das Gemisch im selben Temperaturbereich zur Vervollständigung der Reaktion noch 15 Minuten bis 2 Stunden, vorzugsweise 15 Minuten bis eine Stunde belassen oder vorzugsweise rühren.

Die Herstellung des in dem erfindungsgemäßen Verfahren eingesetzten Diphenylmagnesiums erfolgt in an sich bekannter Weise. Beispielsweise kann man ein Phenylmagnesiumhalogenid, z. B. Phenylmagnesiumchlorid, in einem geeigneten Lösungsmittel mit Dioxan versetzen, wobei unter Verschiebung des Schlenk-Gleichgewichts Diphenylmagnesium und der entsprechende Magnesiumhalogenid-Dioxan-Komplex entstehen. Letzterer fällt in der Regel aus, wird aber bevorzugt nicht aus der Lösung entfernt. Geeignete Lösungsmittel sind im Allgemeinen acyclische und cyclische Ether mit vorzugsweise 4 bis 6 C-Atomen oder deren Mischungen mit aliphatischen, alicyclischen oder aromatischen Kohlenwasserstoffen. Geeignete acyclische Ether sind z. B. Diethylether und tert-Butylmethylether, ein geeigneter cyclischer Ether ist Tetrahydrofuran. Zu den geeigneten aliphatischen bzw. alicyclischen Kohlenwasserstoffen zählen insbesondere n-Hexan bzw. Cyclohexan, geeignete aromatische Kohlenwasserstoffe sind z. B. Benzol, Toluol und Xylole.

Dioxan wird in der Regel mindestens äquimolar im Verhältnis zum Phenylmagnesiumhalogenid eingesetzt. Soll als Phenylmagnesiumverbindung Diphenylmagnesium verwendet werden, so setzt man vorzugsweise Dioxan im Überschuss, beispielsweise in einem Überschuss von 50 bis 500 Mol-%, insbesondere von 100 bis 300 Mol-% und speziell von 100 bis 200 Mol-%, ein. Soll Diphenylmagnesium zuerst in das Phenylmagnesiumalkoxid überführt werden, setzt man vorzugsweise Dioxan und das Phenylmagnesiumhalogenid in einem Molverhältnis im Bereich von 1:1 bis 1,5:1, insbesondere 1:1 bis 1,2:1 und besonders bevorzugt etwa äquimolar ein.

Die Zugabe des Dioxans zu der Lösung des Phenylmagnesiumhalogenids erfolgt in der Regel bei einer Temperatur im Bereich von -20 bis 60 °C, vorzugsweise im Bereich von -10 bis 40 °C.

Üblicherweise belässt man die nach Zugabe des Dioxans erhaltene Mischung noch 15 Minuten bis 2 Stunden, vorzugsweise 20 Minuten bis eine Stunde, in dem für die Zugabe des Dioxans genannten Temperaturbereich, bevor man sie ins erfindungsgemäße Verfahren einsetzt.

Sowohl die Herstellung von Diphenylmagnesium, die Umsetzung zum Phenylmagnesiumalkoxid als auch die Umsetzung der Phenylmagnesiumverbindung mit dem Propanon II nach Grignard erfolgen geeigneterweise unter einer Inertgasatmosphäre. Als Inertgase kommen beispielsweise Stickstoff und die Edelgase wie Argon sowie deren Mischungen in Betracht.

In der Grignard-Reaktion des Propanons II mit der Phenylmagnesiumverbindung setzt man in der Regel die Phenylmagnesiumverbindung und das Propanon II in einem Molverhältnis im Bereich von 0,8:1 bis 3:1, vorzugsweise von 1:1 bis 3:1 ein. Im Falle der Verwendung von Diphenylmagnesium oder des Phenylmagnesiumalkoxids setzt man die Phenylmagnesiumverbindung und das Propanon II besonders bevorzugt in einem Molverhältnis im Bereich von 1:1 bis 2:1, insbesondere von 1:1 bis 1,3:1, ein.

In der Regel legt man die Phenylmagnesiumverbindung in Form einer Lösung in einem der oben genannten, für Grignard-Reaktionen geeigneten, organischen Lösungsmittel vor und fügt das Propanon II bei einer Temperatur im Bereich von -20 °C bis zur Siedetemperatur, vorzugsweise im Bereich von -10 °C bis 90 °C und besonders bevorzugt im Bereich von 0 °C bis 70 °C hinzu. Dabei wird die Phenylmagnesiumverbindung in der Regel in einer Konzentration im Bereich von 0,1 bis 10 mol/l, vorzugsweise im Bereich von 0,1 bis 3 mol/l und besonders bevorzugt im Bereich von 0,2 bis 2 mol/l eingesetzt.

Die Zugabe des Propanons II kann in einer Portion oder vorzugsweise über einen Zeitraum von wenigen Minuten bis zu mehreren Stunden, z. B. 5 Minuten bis 5 Stunden, erfolgen. Die Zugabe des Propanons II erfolgt entweder in Form einer Lösung in einem der oben genannten, für Grignard-Reaktionen geeigneten, inerten Lösungsmittel oder vorzugsweise in Reinform. Bei der Zugabe als Lösung beträgt die Konzentration des Propanons II in der Regel 0,1 bis 20 mol/1, vorzugsweise 1 bis 15 mol/1. Zur Vervollständigung der Reaktion wird das Reaktionsgemisch üblicherweise 15 Minuten bis 5 Stunden, speziell 30 Minuten bis 2 Stunden bei einer Temperatur im Bereich von -20 °C bis zur Siedetemperatur des Reaktionsgemischs, vorzugsweise im Bereich von -10 °C bis 90°C und besonders bevorzugt im Bereich von 10 °C bis 80 °C belassen, wobei man vorzugsweise zur besseren Durchmischung rührt. Die Aufarbeitung erfolgt wie für Grignard-Reaktionen üblich wässrig-extraktiv, z. B. indem man das Reaktionsgemisch mit Wasser, einer wässrigen Ammoniumchloridlösung oder einer sauren wässrigen Lösung quencht, wobei man im letzten Fall den pH-Wert des entstandenen Gemischs anschließend alkalisch einstellt, das gequenchte Gemisch gegebenenfalls nach Abtrennung einer organischen Phase mit einem mit Wasser nicht mischbaren, zum Lösen des Produkts geeigneten Lösungsmittel extrahiert und aus dem Extrakt bzw. aus dem mit der organischen Phase vereinigten Extrakt das Lösungsmittel entfernt. Geeignete Lösungsmittel sind beispielsweise Aromaten wie Benzol oder Toluol, die oben genannten acyclischen Ether, Ester wie Ethylacetat oder chlorhaltige Aliphaten wie Dichlor- oder Trichlormethan.

Das aus der Umsetzung des Propanons II mit einer Phenylmagnesiumverbindung erhaltene Rohprodukt besteht im Wesentlichen aus den vier diastereomeren Enantiomerenpaaren Ia bis Id des Aminopropanols I, wobei das Enantiomerenpaar Ia (Biperiden) mit in der Regel wenigstens 50 % die Hauptmenge bildet.

Zur Isolierung des Biperidens (Ia) aus dem Diastereomerengemisch wird dieses unter Erwärmen, vorzugsweise bei einer Temperatur von 40 bis 80 °C, insbesondere von 50 bis 70 °C, in einem Gemisch aus Wasser und einem polaren, mit Wasser mischbaren organischen Lösungsmittel gelöst. Geeignete Lösungsmittel sind C₁-C₃-Alkanole, d. h. Methanol, Ethanol, n-Propanol und Isopropanol. Vorzugsweise verwendet man wässriges Isopropanol, besonders bevorzugt 70 bis 95%iges Isopropanol und insbesondere 90%iges Isopropanol. Die hier und im Folgenden gemachten %-Angaben bezüglich des Isopropanolgehalts beziehen sich auf das Volumen des Isopropanols bezogen auf das Gesamtvolumen des wasserhaltigen Lösungsmittels. Zu dieser Lösung wird HC1, beispielsweise in Form einer Lösung von Chlorwasserstoff in einem organischen Lösungsmittel, vorzugsweise in einem der genannten C₁-C₃-Alkanole, bevorzugt in Isopropanol, oder in Form von Salzsäure, zugegeben. HCl wird wenigstens äquimolar bezogen auf den Aminoalkohol I, vorzugsweise in einem Überschuss von 5 bis 50 Mol-% und besonders bevorzugt von 5 bis 20 Mol-%, eingesetzt. Die Zugabe erfolgt vorzugsweise bei erhöhter Temperatur, z. B. bei 40 bis 80 °C und insbesondere bei 50 bis 70 °C. Zur Vervollständigung der Reaktion wird nach vollendeter Zugabe das Reaktionsgemisch bei einer Temperatur von 50 °C bis zur Siedetemperatur des Reaktionsgemischs 0,5 bis 3 Stunden belassen, wobei man vorzugsweise rührt. In einer bevorzugten Ausführungsform wird das Reaktionsgemisch zuerst zwei Drittel der Zeit bei 55 bis 65 °C und anschließend ein Drittel der Zeit bei Rückflusstemperatur gerührt. Das Reaktionsgemisch wird anschließend auf eine Temperatur im Bereich von 0 bis 30°C abgekühlt, gegebenenfalls noch bis zu mehreren Stunden, z. B. bis zu 10 Stunden, vorzugsweise bis zu 5 Stunden, in diesem Temperaturbereich gerührt und dann das gebildete Hydrochlorid in üblicher Weise von der Lösung abgetrennt.

Zur weiteren Aufreinigung des Hydrochlorids wird dieses im Allgemeinen feucht oder trocken in Wasser und einer ausreichenden Menge eines oder mehrerer polarer, mit Wasser begrenzt oder nicht mischbarer Dialkylether mit 4 bis 8 C-Atomen wie Diethylether, tert-Butylmethylether und insbesondere Diisopropylether aufgenommen und das Gemisch mit einer geeigneten Base versetzt. Ausreichende Mengen an organischen Lösungsmitteln sind z. B. 4 bis 10 ml Lösungsmittel pro Gramm trockenen Hydrochlorids. Wasser und organisches Lösungsmittel werden vorzugsweise in einem Volumenverhältnis im Bereich von 1:2 bis 1:5 eingesetzt.

Geeignete Basen sind Alkali- und Erdalkalihydroxide sowie Alkalicarbonate; besonders bevorzugt werden Natrium- oder Kaliumhydroxid bzw. ihre wässrigen Lösungen und insbesondere Natriumhydroxid bzw. Natronlauge verwendet. Möglich ist aber auch die Verwendung wasserlöslicher organischer Basen, beispielsweise aliphatisch substituierte Amine mit 2 bis 8 C-Atomen. Die Base wird wenigstens äquimolar, vorzugsweise im Überschuss, insbesondere in einem Überschuss von 5 bis 15 Mol-%, bezogen auf das Hydrochlorid, eingesetzt.

Erfindungsgemäß erfolgt die Umsetzung mit der Base in der wärme. Hierzu wird vor, während oder bevorzugt nach Zugabe der Base die Mischung auf eine Temperatur im Bereich oberhalb 25°C bis zur Siedetemperatur des Reaktionsgemischs, vorzugsweise im Bereich von 30 bis 70 °C, bei Verwendung von Diisopropylether als Dialkylether vorzugsweise im Bereich von 40 bis 65 °C, insbesondere von 55 bis 60 °C, erwärmt. Hierbei entstehen in der Regel zwei klare Phasen, die in der Wärme, d. h. oberhalb 25°C, vorzugsweise 30-70°C, im Falle der Verwendung von Diisopropylether als Dialkylether im oben erwähnten Temperaturbereich, getrennt werden. Die organische Phase wird in der Wärme, d. h. oberhalb 25°C, vorzugsweise 30-70°C, im Falle der Verwendung von Diisopropylether als Dialkylether im oben erwähnten Temperaturbereich, mit Wasser gewaschen und dann vorzugsweise bei Normaldruck durch Entfernen des Lösungsmittels bis zu einem Gewicht/Volumen-Verhältnis des Pro-dukts zum Lösungsmittel im Bereich von 1:2 bis 1:6, vorzugsweise von 1:3 bis 1:4,5, aufkonzentriert. Beim Abkühlen des Gemischs auf Raumtemperatur oder darunter, vorzugsweise jedoch nicht unter -10 °C, kristallisiert sauberes Biperiden (Ia) aus, das nach üblichen Methoden der Feststoffisolierung, z. B. Abfiltrieren des Feststoffs oder Dekantieren der Mutterlauge, gewonnen wird.

Das durch die erfindungsgemäße Aufreinigung erhältliche Biperiden (Ia) ist insbesondere bei Einsatz von Diphenylmagnesium oder des Phenylmagnesiumalkoxids in einer höheren Ausbeute, in einem höhe-ren Reinheitsgrad und in weniger Schritten als nach üblichen Verfahren erhältlich. Der Reinheitsgrad beträgt in der Regel wenigstens 99,0 % oder besser.

Biperiden (Ia) kann anschließend mit einer pharmakologisch ver-träglichen Säure in üblicher Weise in sein Säureadditionssalz überführt werden. Geeignete Säuren sind beispielsweise Halogenwasserstoffsäuren, insbesondere Chlorwasserstoff bzw. Salzsäure, sowie organische Mono- oder Dicarbonsäuren wie Essigsäure, Oxalsäure, Maleinsäure, Fumarsäure, Milchsäure, Weinsäure, Adipinsäure oder Benzoesäure, weiterhin Phosphorsäure und Schwefelsäure sowie die in "Fortschritte der Arzneimittelforschung, Band 10, S. 224ff, Birkhäuser Verlag, Basel, Stuttgart, 1966" genannten Säuren. Üblicherweise kommt Biperiden (Ia) als Hydrochlorid in den Handel.

Das in die Grignard-Reaktion eingesetzte 1-(Bicyclo[2.2.1]hept-5-en-2-yl)-3-piperidino-1-propanon (II) erhält man durch Umsetzung von exo-1-(Bicyclo[2.2.1]hept-5-en-2-yl)ethanon (III-exo) in einer Reaktion nach Mannich in Anwesenheit einer Säure mit Piperidin und einer Formaldehydquelle oder mit dem Additionsprodukt von Piperidin und Formaldehyd, vorzugsweise in einem geeigneten Lösungsmittel.

Unter exo-1-(Bicyclo[2.2.1]hept-5-en-2-yl)ethanon (III-exo) soll im Folgenden ein Ethanon III verstanden werden, das zu wenigstens 96 %, vorzugsweise zu wenigstens 97 % und besonders bevorzugt zu wenigstens 98 %, aus dem exo-Isomeren III-exo besteht.

Geeignete Lösungsmittel sind insbesondere C₁-C₄-Alkanole, z. B. Methanol, Ethanol, n-Propanol, Isopropanol, sek-Butanol und Isobutanol. Vorzugsweise wird Isopropanol verwendet. Das exo-Ethanon (III-exo) und Piperidin werden in der Regel in einem Molverhältnis im Bereich von 0,5:1 bis 1,5:1, vorzugsweise 1:1 eingesetzt. Formaldehyd liegt in der Regel im Überschuss vor, wobei der Überschuss bis zu 100 Mol-%, bezogen auf Piperidin, insbesondere bis zu 50 Mol-% betragen kann. Formaldehyd kann dabei sowohl gasförmig, als Formalin, als Trioxan als auch als Paraformaldehyd eingesetzt werden. Bevorzugt verwendet man Paraformaldehyd insbesondere in Kombination mit Piperidiniumhydrochlorid. In einer bevorzugten Vorgehensweise werden das exo-Ethanon (III-exo), Piperidinhydrochlorid und Paraformaldehyd in Molverhältnissen von 1:0,9-1,2:1-1,4 miteinander umgesetzt. Als Lösungsmittel verwendet man dann vorzugsweise ein C₁-C₄-Alkanol, insbesondere Isopropanol. Die Reaktionstemperatur liegt in der Regel im Bereich von 10 °C bis zur Siedetemperatur des Gemischs. Vorzugsweise erhitzt man zum Rückfluss.

Die Aufarbeitung erfolgt in an sich bekannter Weise. Hierzu wird man gewöhnlich zuerst das Lösungsmittel bei vermindertem Druck entfernen und den Rückstand in Wasser aufnehmen. Die hierbei erhaltene wässrige Lösung wird man mit einem geeigneten organischen Lösungsmittel, d. h. mit einem mit Wasser nicht mischbaren, mäßig polaren Lösungsmittel, beispielsweise einem aliphatischen Ether mit 4 bis 6 C-Atomen, wie Diethylether, tert-Butylmethylether oder vorzugsweise Diisopropylether, extrahieren. Diese Extraktion erfolgt in der Regel bei pH ≤ 7 und dient der Entfernung von Nebenprodukten. Insbesondere wird man die zunächst saure Lösung extrahieren, dann den pH-Wert der wässrigen Phase durch Zugabe geringer Mengen an Base abstumpfen und erneut extrahieren, wobei ein pH ≤ 7 eingehalten wird. Dann wird man vorzugsweise die wässrige Phase durch Zugabe von Base in ein oder mehreren Stufen alkalisch stellen, vorzugsweise auf pH ≥ 7,5 insbesondere pH 7,5 bis 9 und speziell pH 8,0 bis 8,5, um das 1-(Bicyclo[2.2.1]-hept-5-en-2-yl)-3-piperidino-1-propanon (II), das noch als Säureadditionssalz vorliegt, in das freie Amin zu überführen. Als Basen kommen hierfür die üblichen anorganischen Basen in Betracht wie KOH, NaOH, Na₂CO₃, K₂CO₃ und dergleichen. Die wässrige Phase wird dann mit einem der oben genannten, mit Wasser nicht mischbaren, mäßig polaren Lösungsmittel, vorzugsweise Diisopropylether, ein- oder mehrfach extrahiert. Zur Gewinnung des Propanons II aus dem Extrakt wird das Lösungsmittel gegebenenfalls unter vermindertem Druck entfernt. Zur weiteren Reinigung kann der Rückstand über eine Vakuumdestillation bei einem Druck von vorzugsweise weniger als 10 mbar, besonders bevorzugt von weniger als 5 mbar und insbesondere von weniger als 1 mbar, gereinigt werden. Das erhaltene Gemisch besteht aus exo- und endo-1-(Bicyclo-[2.2.1]hept-5-en-2-yl)-3-piperidino-l-propanon (II) in einem Verhältnis von wenigstens 2,5:1, vorzugsweise von wenigstens 3,0:1 und insbesondere von 3,5-4,0:1.

Ein bevorzugtes Verfahren zur Herstellung des Propanons II-exo, insbesondere die Aufarbeitung des bei der Umsetzung von III-exo mit Piperidin und Formaldehyd nach Mannich erhaltenen Produkts wird in der parallelen deutschen Patentanmeldung 10124449.5 beschrieben.

Das zur Herstellung des 1-(Bicyclo[2.2.1]hept-5-en-2-yl)-3-piperidino-1-propanons (II) verwendete exo-1-(Bicyclo[2.2.1]hept-5-en-2-yl)ethanon (III-exo) erhält man durch die Umsetzung von Cyclopentadien und Methylvinylketon in einer Cycloaddition nach Diels und Alder. Ein bevorzugtes Verfahren zur Herstellung von III, das ein Produkt mit einem hohen Anteil an III-exo liefert, wird in der parallelen deutschen Patentanmeldung 10124450.9 beschrieben. Die Cycloaddition kann in einem für solche Reaktionen gängigen Lösungsmittel wie Diethylether, Benzol, Toluol oder Xylol oder auch ohne Lösungsmittel durchgeführt werden. Vorzugsweise wird kein Lösungsmittel verwendet. Cyclopentadien und Methylvinylketon werden in der Regel in einem Molverhältnis im Bereich von 3,0:1 bis 0,5:1 eingesetzt. Vorzugsweise werden sie äquimolar oder mit Cyclopentadien im Überschuss umgesetzt, wobei der Überschuss vorzugsweise 50 bis 150 Mol-% beträgt.

Die Reaktion wird in der Regel bei einer Temperatur im Bereich von 0 bis 60 °C, vorzugsweise im Bereich von 20 bis 40 °C durchgeführt.

Niedrig siedende Bestandteile, meist unumgesetzte Edukte, werden in der Regel im Anschluss an die Cycloaddition bei erniedrigtem Druck, vorzugsweise bei 1 bis 150 mbar, destillativ entfernt. Das zurückbleibende Gemisch, das zu etwa 20 % aus exo- und zu etwa 80 % aus endo-1-(Bicyclo[2.2.1]hept-5-en-2-yl)ethanon besteht, wird mit einem Alkali-C₁-C₄-alkoholat umgesetzt. Die Menge an Alkalialkoholat beträgt in der Regel 0,1 bis 5 Gew.-%, vorzugsweise 0,2 bis 2 Gew.-%, bezogen auf das Gesamtgewicht der Mischung. Vorzugsweise wird Natriummethanolat verwendet. Die zur Isomerisierung des Ethanons III erforderliche Temperatur liegt in der Regel im Bereich von 50 bis 110 °C, vorzugsweise im Bereich von 60 bis 100 °C. Hierzu erhitzt man häufig die Mischung unter vermindertem Druck zum Rückfluss, vorzugsweise bei einem Druck von 1 bis 100 mbar und insbesondere bei einem Druck von 5 bis 50 mbar. Man wendet diese Bedingungen in der Regel 10 Minuten bis 5 Stunden, insbesondere 20 Minuten bis 3 Stunden und speziell 0,5 Stunden bis 2 Stunden an und beginnt dann das erhaltene Gemisch fraktioniert zu destillieren, wobei bevorzugt das exo-Isomer von III abdestilliert. Man geht davon aus, dass durch das Entfernen des exo-Isomeren aus dem Gleichgewicht die Isomerisierung des endo-Ethanons zur exo-Form gefördert wird. Die fraktionierte Destillation erfolgt in der Regel über eine Kolonne unter vermindertem Druck, vorzugsweise im Bereich von 1 bis 100 mbar, insbesondere von 1 bis 50 und speziell von 1 bis 20 mbar. Die Destillationstemperatur (Kopftemperatur) wird vorzugsweise auf 50 bis 100 °C und speziell auf 50 bis 80 °C eingestellt. Auf diese Weise erhält man exo-1-(Bicyclo[2.2.1]hept-5-en-2-yl)ethanon (III-exo) in einem Reinheitsgrad, der wenigstens 96 % beträgt. Durch Redestillation des Destillats erhält man das exo-Ethanon III-exo mit einem Reinheitsgrad von bis zu 100 %.

Das folgende Beispiel dient der Veranschaulichung der Erfindung.

### Beispiel

### 1. Herstellung des Ausgangsmaterials

### 1.1 exo-1-(Bicyclo[2.2.1]hept-5-en-2-yl)ethanon (III-exo)

Zu 210,3 g Methylvinylketon wurden zügig 198,3 g Cyclopentadien gegeben. Nach beendeter Zugabe wurde die Reaktionslösung noch eine Stunde bei Raumtemperatur nachgerührt und dann bei einer Temperatur von 58°C und einem Druck von 20 mbar nicht umgesetztes Edukt destillativ entfernt. Der Eindampfrückstand, hauptsächlich bestehend aus einem Gemisch aus der exo- und der endo-Form von 1-(Bicyclo[2.2.1]hept-5-en-2-yl)ethanon (III) im Verhältnis von 1:4, wurde mit 5 g Natriummethanolat versetzt und eine Stunde bei einem Druck von 10 bis 20 mbar zum Rückfluss erhitzt. Das Reaktionsgemisch wurde anschließend über eine Kolonne bei einer Temperatur von 75 °C und einem Druck von 20 mbar destilliert. Erhalten wurden dabei 298,3 g (73 % der Theorie) exo-1-(Bicyclo[2.2.1]hept-5-en-2-yl)ethanon (III-exo) in Form eines leicht gelblichen Öls.

### 1.2 1-(Bicyclo[2.2.1]hept-5-en-2-yl)-3-piperidino-1-propanon (II)

68,1 g exo-1-(Bicyclo[2.2.1]hept-5-en-2-yl)ethanon (III-exo), 60,8 g Piperidinhydrochlorid und 18 g Paraformaldehyd wurden in 140 ml Isopropanol fünf Stunden zum Rückfluss erhitzt. Das Lösungsmittel wurde im Vakuum entfernt und der Rückstand in 100 ml Wasser aufgenommen. Die Lösung wurde drei Mal mit jeweils 50 ml Diisopropylether gewaschen und dann mit 50%iger Natronlauge auf pH 10 eingestellt. Es wurde drei Mal mit jeweils 50 ml Diisopropylether extrahiert, die drei Extrakte vereinigt und das Lösungsmittel am Rotationsverdampfer entfernt. Der Eindampfrückstand wurde im Kugelrohr bei 75 °C im Hochvakuum bei 0,001 mbar destilliert. Als Destillat wurden 50,2 g (43 % der Theorie) eines Gemischs aus exo- und endo-1-(Bicyclo[2.2.1]hept-5-en-2-yl)-3-piperidino-1-propanon (II) im Verhältnis von 3,5:1 in Form eines farblosen Öls erhalten.

### 2. Herstellung von Biperiden (Ia)

Zu 1500 g einer auf 0 °C abgekühlten, 25%igen Lösung von Phenylmagnesiumchlorid (375 g, 2,74 mol) in Tetrahydrofuran wurden unter Eisbadkühlung 603,6 g (6,85 mol) Dioxan gegeben, wobei sich ein weißer Niederschlag bildete. Es wurde noch 30 Minuten unter Eisbadkühlung gerührt und anschließend unter Eisbadkühlung 320 g (1,37 mol) des 3,5:1-Gemischs aus den exo- und endo-Formen von 1-(Bicyclo[2.2.1]hept-5-en-2-yl)-3-piperidino-1-propanon (II) hinzugefügt. Nach beendeter Zugabe wurde das Eisbad entfernt und noch eine Stunde bei Raumtemperatur gerührt. Die Lösung wurde anschließend langsam zu 1500 ml eiskaltem Wasser gegeben und dann drei Mal mit jeweils 500 ml Toluol extrahiert. Die organischen Phasen wurden vereinigt, über Natriumsulfat getrocknet und am Rotationsverdampfer eingedampft. Der Eindampfrückstand, 433,8 g eines Gemischs, das im Wesentlichen aus den Formen Ia bis Id von 1-(Bicyclo[2.2.1]hept-5-en-2-yl)-1-phenyl-3-piperidino-1-propanol (I) im Verhältnis (GC) von 10,4:3,4:3,0:1 bestand, wurde in 3500 ml 90%igem Isopropanol heiß gelöst und die Lösung bei 60°C mit 228 ml einer 6-molaren Lösung von Chlorwasserstoff in Isopropanol versetzt. Nach der Säurezugabe wurde eine Stunde bei 60 °C und dann noch 0,5 Stunden bei Rückflusstemperatur nachgerührt. Nach dem Abkühlen auf Raumtemperatur wurden die ausgefallenen Kristalle abgetrennt und zwei Mal mit jeweils 200 ml Isopropanol gewaschen. Das so erhaltene feuchte Hydrochlorid wurde in 1150 ml Diisopropylether und 350 ml Wasser unter Rühren mit 135 ml 5M Natronlauge versetzt. Das Gemisch wurde auf 55°C erwärmt und dann bei dieser Temperatur die Wasserphase abgetrennt und die Diisopropylether-Lösung zwei Mal mit jeweils 200 ml Wasser gewaschen. Aus der gewaschenen Diisopropylether-Lösung wurden bei Normaldruck 500 ml Lösungsmittel destillativ entfernt. Man ließ den Destillationsrückstand unter Rühren abkühlen. Anschließend wurde weiter auf 20 °C abgekühlt, eine Stunde bei dieser Temperatur gerührt und dann die ausgefallenen Kristalle abgetrennt, mit 50 ml Diisopropylether gewaschen und im Vakuum bei 50 °C getrocknet. Erhalten wurden 118 g Biperiden (Ia) als farblose Kristalle vom Schmelzpunkt 112 bis 114 °C (Ullmanns Enzyklopädie der techn. Chemie, 4. Aufl., Band 21, Verlag Chemie, 1982, S. 627: 112-114 °C); das sind 28 % der Theorie.

### 3. Herstellung des Biperidenhydrochlorids

6,7 g Biperiden (Ia) wurden in 75 ml Isopropanol durch Erwärmen auf Rückflusstemperatur gelöst. Die Lösung wurde heiß filtriert und das Filter mit 7 ml Isopropanol nachgewaschen. Die vereinigten Filtrate wurden bei 75 °C mit 4,7 ml 5-molarer Salzsäure versetzt. Anschließend wurde das Gemisch noch 15 Minuten zum Rückfluss erhitzt. Nach dem Abkühlen auf Raumtemperatur wurde die ausgefallene Festsubstanz abgesaugt, mit 7 ml Isopropanol gewaschen und im Vakuum bei 70 °C getrocknet. Erhalten wurden 7,3 g Biperidenhydrochlorid in Form farbloser Kristalle vom Schmelzpunkt 278 bis 280 °C (Ullmanns Enzyklopädie der techn. Chemie, 4. Aufl., Band 21, Verlag Chemie, 1982, S. 627: 278 - 280 °C); das sind 98 % der Theorie.

## Patentansprüche

1. Verfahren zur Herstellung von Biperiden der Formel (Ia) 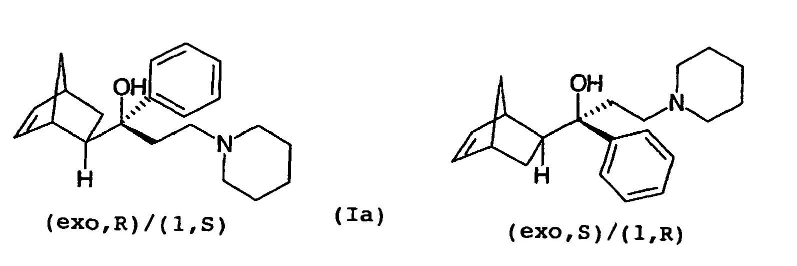 durch Umsetzung eines exo/endo-Gemischs von 1-(Bicyclo[2.2.1]hept-5-en-2-yl)-3-piperidino-1-propanon (II) mit einem exo/endo-Verhältnis von wenigstens 2,5:1 mit einer Phenylmagnesiumverbindung zu einem Biperiden enthaltenden Isomerengemisch von I-(Bicyclo[2.2.1]-hept-5-en-2-yl)-1-phenyl-3-piperidino-1-propanol (I), **dadurch gekennzeichnet, dass** die Isolierung des Biperidens (Ia) aus dem Isomerengemisch folgende Schritte umfasst:
a) Umsetzung des Isomerengemischs mit HCl in einem Gemisch aus Wasser und einem polaren, mit Wasser mischbaren organischen Lösungsmittel und Isolierung des dabei gebildeten Hydrochlorids,
b) Umsetzung des Hydrochlorids in einer Mischung aus Wasser und wenigstens einem polaren, mit Wasser begrenzt oder nicht mischbaren Dialkylether mit 4 bis 8 C-Atomen mit einer Base unter Erwärmen,
c) Trennen der zwei gebildeten Phasen in der Wärme,
d) Verdampfen eines Teils des Ethers aus der organischen Phase und
e) Kristallisieren des Biperidens (Ia) durch Abkühlen.

2. Verfahren nach Anspruch 1, **dadurch gekennzeichnet, dass** man in Schritt a) wässriges Isopropanol als Lösungsmittelgemisch verwendet.

3. Verfahren nach einem der vorhergehenden Ansprüche, **dadurch gekennzeichnet, dass** man in Schritt a) das Isomerengemisch mit HCl (Chlorwasserstoff oder Salzsäure) bei einer Temperatur im Bereich von 40 bis 80 °C umsetzt.

4. Verfahren nach einem der vorhergehenden Ansprüche, **dadurch gekennzeichnet, dass** man in Schritt a) das Hydrochlorid bei einer Temperatur im Bereich von 0 °C bis 30 °C isoliert.

5. Verfahren nach einem der vorhergehenden Ansprüche, **dadurch gekennzeichnet, dass** man in Schritt b) Diisopropylether als organisches Lösungsmittel verwendet.

6. Verfahren nach einem der vorhergehenden Ansprüche, **dadurch gekennzeichnet, dass** man in Schritt b) als Base ein Alkali- oder Erdalkalihydroxid oder ein Alkalicarbonat oder eine wasserlösliche organische Base verwendet.

7. Verfahren nach einem der vorhergehenden Ansprüche, **dadurch gekennzeichnet, dass** man in Schritt d) den Ether verdampft, bis das Gewicht/Volumen-Verhältnis von Produkt zum Ether im Bereich von 1:2 bis 1:6 liegt.

8. Verfahren nach einem der vorhergehenden Ansprüche, **dadurch gekennzeichnet, dass** man als Phenylmagnesiumverbindung ein Phenylmagnesiumhalogenid, Diphenylmagnesium oder ein Phenylmagnesiumalkoxid der allgemeinen Formel IV 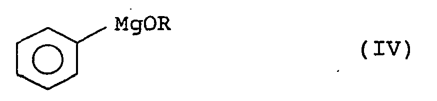 einsetzt, wobei R für C₁-C₄-Alkyl, C₄-C₆-Cycloalkyl, C₄-C₆-Cycloalkyl-C₁-C₄-alkyl, Phenyl-C₁-C₄-alkyl, substituiertes Phenyl-C₁-C₄-alkyl, Heteroaryl, Heteroaryl-C₁-C₄-alkyl oder Benzhydryl steht.

9. Verfahren nach Anspruch 8, **dadurch gekennzeichnet, dass** man als Phenylmagnesiumverbindung Diphenylmagnesium oder das Phenylmagnesiumalkoxid der Formel IV einsetzt.

10. Verfahren nach Anspruch 9, **dadurch gekennzeichnet, dass** man das Phenylmagnesiumalkoxid der Formel IV herstellt, indem man Diphenylmagnesium mit einem Alkohol der allgemeinen Formel ROH umsetzt, wobei R die in Anspruch 8 angegebenen Bedeutungen besitzt.

11. Verfahren nach einem der Ansprüche 9 oder 10, **dadurch gekennzeichnet, dass** man Diphenylmagnesium herstellt, indem man ein Phenylmagnesiumhalogenid mit Dioxan umsetzt und den gleichzeitig gebildeten Magnesiumhalogenid-Dioxan-Komplex nicht abtrennt.

## Claims

1. Process for the preparation of biperiden 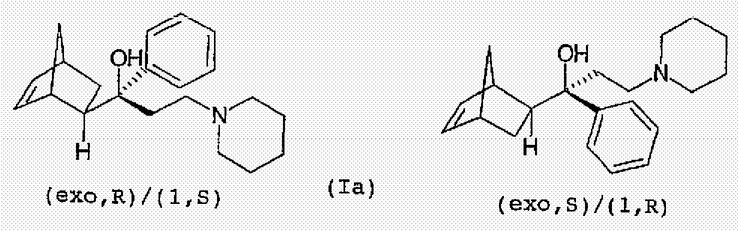 by reaction of an exo/endo mixture of 1-(bicyclo[2.2.1]hept-5-en-2-yl)-3-piperidino-1-propanone (II) having an exo/endo ratio of at least 2.5 : 1 with a phenylmagnesium compound to give a biperiden-containing isomer mixture of 1-(bicyclo[2.2.1]hept-5-en-2-yl)-1-phenyl-3-piperidino-1-propanol (I), **characterized in that** the isolation of biperiden (la) from the isomer mixture comprises the following steps:
a) reaction of the isomer mixture with HCl in a mixture of water and a polar, water-miscible organic solvent, and isolation of the hydrochloride formed,
b) reaction of the hydrochloride in a mixture of water and at least one polar, sparingly water-miscible or -immiscible dialkyl ether having 4 to 8 C atoms with a base with warming,
c) separation of the two phases formed at elevated temperature,
d) evaporation of some of the ether from the organic phase, and
e) crystallization of the biperiden (Ia) by cooling.

2. Process according to Claim 1, **characterized in that** aqueous isopropanol is used as solvent mixture in step a).

3. Process according to one of the preceding claims, **characterized in that** the isomer mixture is reacted with HCl (hydrogen chloride or hydrochloric acid) in step a) at a temperature in the range from 40 to 80°C.

4. Process according to one of the preceding claims, **characterized in that** the hydrochloride is isolated in step a) at a temperature in the range from 0°C to 30°C.

5. Process according to one of the preceding claims, **characterized in that** diisopropyl ether is used as organic solvent in step b).

6. Process according to one of the preceding claims, **characterized in that** the base used in step b) is an alkali or alkaline earth metal hydroxide or an alkali metal carbonate or a water-soluble organic base.

7. Process according to one of the preceding claims, **characterized in that** the ether is evaporated in step d) until the weight/volume ratio of product to the ether is in the range from 1 : 2 to 1 : 6.

8. Process according to one of the preceding claims, **characterized in that** the phenylmagnesium compound employed is a phenylmagnesium halide, diphenylmagnesium or a phenylmagnesium alkoxide of the general formula IV 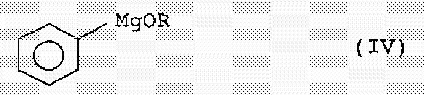 where R stands for C₁-C₄-alkyl, C₄-C₆-cycloalkyl, C₄-C₆-cydoalkyl-C₁-C₄-alkyl, phenyl-C₁-C₄-alkyl, substituted phenyl-C₁-C₄-alkyl, heteroaryl, heteroaryl-C₁-C₄-alkyl or benzhydryl.

9. Process according to Claim 8, **characterized in that** the phenylmagnesium compound employed is diphenylmagnesium or the phenylmagnesium alkoxide of the formula IV.

10. Process according to Claim 9, **characterized in that** the phenylmagnesium alkoxide of the formula IV is prepared by reacting diphenylmagnesium with an alcohol of the general formula ROH, where R has the meanings indicated in Claim 8.

11. Process according to one of Claims 9 or 10, **characterized in that** diphenylmagnesium is prepared by reacting a phenylmagnesium halide with dioxane, and the magnesium halide/dioxane complex formed at the same time is not separated off.

## Revendications

1. Procédé pour la préparation de bipéridène de formule (Ia) : 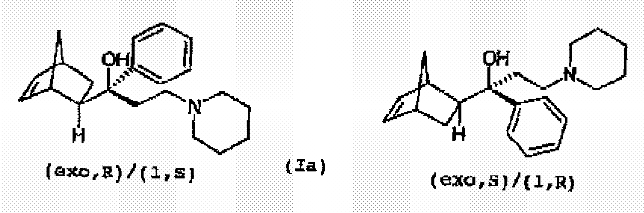 en transformant un mélange endo/exo de 1-(bicyclo[2.2.1]hept-5-èn-2-yl)-3-pipéridino-1-propanone (II) selon un rapport endo/exo d'au moins 2,5/1 avec un composé de phénylmagnésium en un mélange d'isomères de 1-(bicyclo[2.2.1]hept-5-èn-2-yl)-1-phényl-3-pipéridino-1-propanol (I) contenant du bipéridène, **caractérisé en ce que** l'isolement du bipéridène (Ia) à partir du mélange d'isomères comprend les étapes suivantes :
a) la transformation du mélange d'isomères avec du HCl dans un mélange d'eau et d'un solvant organique polaire miscible à l'eau et l'isolement du chlorhydrate ainsi formé ;
b) la transformation du chlorhydrate par chauffage, avec une base, dans un mélange d'eau et d'au moins un éther dialkylique polaire miscible à l'eau de façon limitée ou immiscible à l'eau ayant 4 à 8 atomes de carbone,
c) la séparation des deux phases formées à la chaleur,
d) l'évaporation d'une partie de l'éther à partir de la phase organique et
e) la cristallisation du bipéridène (Ia) par refroidissement.

2. Procédé selon la revendication 1, **caractérisé en ce qu'**on utilise à l'étape a) de l'isopropanol aqueux comme mélange solvant.

3. Procédé selon l'une des revendications précédentes, **caractérisé en ce qu'**on transforme à l'étape a) le mélange d'isomères avec du HCl (chlorure d'hydrogène ou acide chlorhydrique) à une température dans la plage de 40 à 80 °C.

4. Procédé selon l'une des revendications précédentes, **caractérisé en ce qu'**on isole à l'étape a) le chlorhydrate à une température dans la plage de 0 °C à 30 °C.

5. Procédé selon l'une des revendications précédentes, **caractérisé en ce qu'**on utilise à l'étape b) l'éther diisopropylique comme solvant organique.

6. Procédé selon l'une des revendications précédentes, **caractérisé en ce qu'**on utilise comme base à l'étape b) un hydroxyde alcalin ou un hydroxyde alcalino-terreux, un carbonate alcalin ou une base organique hydrosoluble.

7. Procédé selon l'une des revendications précédentes, **caractérisé en ce qu'**on évapore l'éther à l'étape d) jusqu'à ce que le rapport poids/volume du produit à l'éther se situe dans la plage de 1/2 à 1/6.

8. Procédé selon l'une des revendications précédentes, **caractérisé en ce qu'**on emploie comme composé de phénylmagnésium un halogénure de phénylmagnésium, du diphénylmagnésium ou un alcoxyde de phénylmagnésium de formule générale IV : 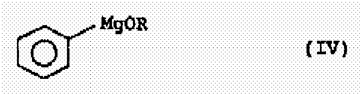 dans laquelle R représente un groupe alkyle en C₁-C₄, un groupe cycloalkyle en C₄-C₆, un groupe cycloalkyle en C₄-C₆-alkyle en C₁-C₄, un groupe phényl (alkyle en C₁-C₄), un groupe phényl(alkyle en C₁-C₄) substitué, un groupe hétéroaryle, un groupe hétéroaryl(alkyle en C₁-C₄) ou un groupe benzhydryle.

9. Procédé selon la revendication 8, **caractérisé en ce qu'**on emploie comme composé de phénylmagnésium du diphénylmagnésium ou l'alcoxyde de phénylmagnésium de formule IV.

10. Procédé selon la revendication 9, **caractérisé en ce qu'**on prépare l'alcoxyde de phénylmagnésium de formule IV en transformant le diphénylmagnésium avec un alcool de formule générale ROH dans laquelle R a la signification donnée à la revendication 8.

11. Procédé selon l'une des revendications 9 ou 10, **caractérisé en ce qu'**on prépare du diphénylmagnésium en transformant un halogénure de phénylmagnésium avec un dioxane et en ne séparant pas le complexe halogénure de phénylmagnésium et dioxane formé en même temps.
